# EUROPEAN PATENT SPECIFICATION

(11) **EP 2 804 940 B1**
(45) Date of publication and mention of the grant of the patent: **19.09.2018**
(21) Application number: 13703739.6
(22) Date of filing: 17.01.2013
(51) Int. Cl.: C11D 7/32, C11D 7/50, A01N 25/02

(54) **USE OF IMPROVED N-ALKYL PYRROLIDONE SOLVENTS**
VERWENDUNG VON N-ALKYL-PYRROLIDONLÖSUNGSMITTELN
UTILISATION DE SOLVANTS AMÉLIORÉS DE TYPE N-ALKYL-PYRROLIDONE

(30) Priority: 17.01.2012 BE 201200037
(43) Date of publication of application: 26.11.2014
(73) Proprietor: Taminco, 9000 Gent (BE)
(72) Inventor: VANDEPUTTE, Bart, B-3191 Schiplaken (BE); MOONEN, Kristof, B-9220 Hamme (BE); ROOSE, Peter, B-9831 Sint-Martens-Latem (BE)
(74) Representative: Gevers Patents
(86) International application number: PCT/EP2013/050852
(87) International publication number: WO 2013/107822

(56) References cited:
- EP-A1- 0 755 987
- WO-A1-2005/090430
- DE-A1- 2 053 104
- DE-A1-102004 012 751
- GB-A- 1 169 113
- US-A- 3 402 178
- US-A- 3 963 560
- ANSELL J M ET AL: "The acute oral toxicity and primary ocular and dermal irritation of selected N-alkyl-2-pyrrolidones", FOOD AND CHEMICAL TOXICOLOGY, PERGAMON, GB, vol. 26, no. 5, 1988, pages 475-479, XP025511639, ISSN: 0278-6915, DOI: 10.1016/0278-6915(88)90060-9 [retrieved on 1988-01-01]
- , 2009, Retrieved from the Internet: URL:http://www.daweichem.cn/template/pro4- 6e.htm [retrieved on 2016-01-18]

## Description

### FIELD OF THE INVENTION

The present invention is concerned with the use of selected pyrrolidones as solvent replacements in specific applications wherein N-methylpyrrolidone (NMP), N-ethyl-2-pyrrolidone (NEP), dimethyl formamide (DMF), N,N-dimethyl acetamide (DMAc), and mixtures thereof, is the appropriate solvent to be used.

### BACKGROUND OF THE INVENTION

More in particular, the invention is concerned with the use of N-n-butylpyrrolidone for the partial or complete replacement of N-methylpyrrolidone (NMP), N-ethyl-2-pyrrolidone (NEP), dimethyl formamide (DMF), N,N-dimethyl acetamide (DMAc) as a solvent in specific applications wherein one of the above mentioned solvents or a mixture thereof is the appropriate solvent to be used.

N-methylpyrrolidone, also called N-methyl-2-pyrrolidone, or 1-methyl-2-pyrrolidone, is a highly polar, aprotic organic solvent with a low viscosity, which is easily miscible with water and other organic solvents and which is used as a common solvent in many applications.

Once regarded as benign, the solvent N-methylpyrrolidone (NMP) is under scrutiny because of concerns over its potential health effects. Although manufacturers say NMP is safe to use when handled properly, these health concerns have opened opportunities for alternative solvents and processes that make do without NMP. The same applies to N-ethyl-2-pyrrolidone (NEP), N,N-dimethyl acetamide (DMAc), and dimethyl formamide (DMF).

Paint makers and other solvent users regarded NMP as something of a wonder chemical during the 1980s and '90s, when they used it to create environmentally friendly polyurethane coatings, paint strippers, and agricultural chemical formulations.

But NMP has increasingly attracted attention as environmental regulators, first in California and more recently in the European Union, have sought to exercise control over the solvent primarily in markets where it represents an inhalation hazard.

Furthermore, NMP is now known to cause reproductive toxicity (it is considered as being reprotoxic) and is being labeled in the EU as "reprotoxic category 2" as from the 1st of December 2010. Formulations containing > 0.3 % of NMP have to be labelled as such. Consequently, the use of the solvent is restricted to professional users. NMP has been placed on the REACH "Substance of Very High Concern" (SVHC) list and will expectedly, sooner or later, be put under authorization or restriction. Therefore, there is a need for NMP to be substituted in many applications on medium term. A similar or even the same problem may present itself for NEP, DMAc and DMF, in particular where these are used as a solvent.

### BACKGROUND PRIOR ART

A number of alternatives for the use of NMP as a solvent have already been disclosed.

WO2005/090447 (BASF, 29 September 2005) discloses the use of N-ethyl-2-pyrrolidone (NEP) as a replacement solvent for NMP. However, this solvent is now listed as reprotoxic in the EU.

WO 2008/012231 (BASF, 31 January 2008) discloses the use of 1,5-dimethylpyrrolidone (DMP) as a replacement solvent for NMP. The application does not provide any toxicological data.. Reprotox screening studies have however indicated that also DMP is suspected as being reprotoxic under the same regulation as NMP.

EP 0 755 987 A1 (CFM GmbH Chemo-technische produkte, 29 January 1997) discloses various compositions for the removal of paint, some of which may comprise a pyrrolidone. Examples 1, 3, 4, 5, 8 and comparative example 2 contain N-methyl pyrrolidone (NMP). Example 7 contains N-propyl pyrrolidone, and Example 2 contains N-octyl pyrrolidone.

WO 2005/090430 A1 (BASF, 29 September 2005) describes the use of N-(cyclo) alkyl pyrrolidones with a (cyclo) alkyl group containing 2 to 6 carbon atoms as solvents in the preparation of polyurethane dispersions. It is further mentioned that cyclohexyl, ethyl, iso-propyl, n-propyl, n-butyl, iso-butyl, sec-butyl and tert-butyl are preferred radicals, ethyl and n-butyl are particular preferred radicals. WO 2005/090430 A1 only refers to toxicity in general and does not refer to non-reprotoxicity at all.

DE 20 53 104 A1 (Gaf Corp, 6 May 1971) describes the use of N-lower-alkyl pyrrolidones, in particular N-methyl pyrrolidone (NMP), as a liquid solvent for absorbing sulfur dioxide from a mixture of gases containing said sulfur dioxide thereby reducing sulfur dioxide in the air. This application demonstrates that N-ethylpyrrolidone, N-tert.-butylpyrrolidone, N-isooctylpyrrolidone and N-isopropyl-piperidone can be used as replacement solvents for N-methyl pyrrolidone for absorbing sulfur dioxide from a mixture of gases containing said sulfur dioxide.

US 3,963,560 (T. S. Mestetsky et al., 15 June 1976) discloses the use of N-alkyllactams, in particular N-alkyl pyrrolidones having N-lower alkyl groups of 1 to 6 carbon atoms, as solvents in the deinking of waste paper. The use of N-t-butyl-pyrrolidone as solvent in a deinking operation for obtaining high foam development has for example been demonstrated.

US 3,402,178 (A.J. Levy et al., 17 September 1968) describes the use of N-alkyl-substituted pyrrolidones, in particular N-methyl pyrrolidone, N-ethyl pyrrolidone and N-isobutyl pyrrolidone, as an inert solvent in a process for the preparation of heterocyclic iminoethers by the reaction of an organic trihalide with an aminoalkanol in the presence of a base. In the working embodiments, use is only demonstrated for N-methyl pyrrolidone as solvent.

WO 2007/115943 A2 (BASF, 18 October 2007) describes the use of N-alkylpyrrolidones in general as organic solvents in a variety of applications.

FR 2001768 discloses the use of N-(lower alkyl)-2-pyrrolidones, in particular N-methyl-2-pyrrolidone, 2- N-ethyl-pyrrolidone and N-n-butyl-2-pyrrolidone and mixtures thereof in a composition containing an active ingredient for increasing absorption of said active ingredient through the skin when applied topically.

Dipropylene glycol dimethyl ether (DPGDME) is commercially offered by Clariant (Basel, Switzerland) as an excellent replacement solvent for formulating polyurethane dispersions as a substitute and alternative to NMP, showing similar solubility properties and similar physical properties. DPGDME based PU dispersions (PUDs) are used for example for leather finishing / coating of car and aircraft upholstery where solvents with low toxicity are mandatory. Mixtures of DPGDME with NMP dissolve DMPA (dimethylol propionic acid) and allow for formulating products with reduced NMP content (Source : website manufacturer).

Vertec BioSolvents, Inc. (Downers Grove, USA) commercially offers a solvent blend with an undisclosed composition, only defined as an ester mixture containing ethyl lactate and a fatty acid methyl ester derived from soya bean oil or corn oil, named ELSOL™ - NMPR, as a replacement solvent blend with renewable, carbon neutral biobased solvents. These biobased solvents are derived from corn, soybeans, citrus fruits and other renewable feedstocks, and allegedly have a reduced toxicity profile (Source : website manufacturer).

Novolyte (East Pleasant Valley Road Independence, USA) commercially offers lower toxicity alternatives for customers looking to replace NMP in coatings and other applications, selected from the group of polyglyme, ethyl diglyme and 1,3-dioxolane. Polyglyme and ethyl diglyme are very stable glycol diethers, while 1,3-dioxolane excells as a small molecule, powerful solubilizing and penetrating solvent in polymer applications. All allegedly share the same aprotic properties of NMP (Source : website manufacturer).

Rhodia (Rhodia SA, Paris, France) commercially offers an allegedly safe and powerful solvent (methyl-5-(dimethylamino)-2-methyl-5-oxopentanoate) for agricultural formulations comprising a combination of ester and amide functions (RhodiaSolv® PolarClean) to replace NMP as a solvent for a number of applications.

Arkema (King of Prussia, USA) offers DMSO (dimethylsulfoxide) as the solvent of choice for formulations in agrochemical, active substances synthesis, electronics, paint stripping, extraction, coatings and cleaning applications.

### SUMMARY OF THE INVENTION

It is the object of this invention to provide alternative solvents for NMP, NEP, DMAc or DMF which are not reprotoxic and which have at least similar, more preferably equal, most preferably better properties for those applications wherein normally NMP, NEP, DMAc or DMF may be used as a solvent.

These alternative solvents for NMP, NEP, DMAc or DMF should preferably have similar physical properties, in particular with regard to viscosity, colour, polarity, reactivity, biodegradability and miscibility with other organic solvents and in particular with water, and should have better toxicological properties, in particular at least be non-reprotoxic.

Surprisingly, these solvents for replacing NMP, NEP, DMAc or DMF may be selected from the group consisting of N-n-butylpyrrolidone.

The present invention therefore provides for the use of N-n-butylpyrrolidone.

In view of the prior art, this finding is surprising as two members of this class of compounds, NMP and NEP, have already shown to be reprotoxic according to to-date criteria. The applicants have found that also the C3 chain lengths are showing similar indications in corresponding screening tests. In terms of chemical structure, it came as a surprise that further elongation of the carbon chain attached to the nitrogen to 4 carbon atoms (N-n-butylpyrrolidone) would yield compounds, suitable as solvent replacements for NMP, NEP, DMAc or DMF and being at least non-reprotoxic.

Therefore, in a first aspect, the invention relates to the use of N-n-butylpyrrolidone.

Non-reprotoxic, in the context of the present invention, means non-reprotoxic following the evaluation according to REGULATION (EC) No 1272/2008 of the European Parliament and of the Council of 16 December 2008, and its amendments up to November 2012.

In a second aspect, the present invention provides for the use of N-n-butylpyrrolidone for the partial or complete replacement of a solvent selected from the list consisting of N-methylpyrrolidone (NMP), N-ethyl-2-pyrrolidone (NEP), dimethyl formamide (DMF), N,N-dimethyl acetamide (DMAc), and mixtures thereof, as a solvent. In this aspect is also included the partial or complete replacement of NMP, NEP, DMAc or DMF in a mixture with one or more other liquid compounds, such as the other compounds that are described as solvents at various places throughout this document.

It is stressed that the invention relates only to the use of N-n-butylpyrrolidone as a solvent in applications where NMP, NEP, DMAc or DMF is suitably used as a solvent. These compounds are particularly favoured because they are high-boiling non-corrosive and polar compounds, and because they are able to dissolve a wide variety of other compounds, and thus are very suitable as solvents. They are also miscible with a wide variety of other solvents including water, ethanol, diethyl ether, chloroform, benzene, ethyl acetate and carbon disulfide.

Such suitability as solvent may be expressed as the ability to dissolve certain compounds at a certain concentration in a stable manner under given circumstances (e.g. temperature). For example, such suitability may be quantitatively expressed, determined or defined by the Hansen solubility parameters (Hansen method). For example, the suitability may be expressed qualitatively more pragmatically (for example as either non-soluble, stable for 7days at room temperature, or stable for 7 days at 0°C) for a list of compounds to be dissolved at a certain concentration, such as one or more of the following compounds in the following concentrations (weight%), which are for example used in agricultural applications: Alachlor 48 %, Propoxur 20 %, Oxyfluorfen 20 %, Difenoconazole 25 %, Trifluralin 40 %, Triadimenol 23 %, Tebuconazole 25 %, Pendimethalin 33 %, Propanil 36 %, Phenmedipham 16 %, Alpha-Cypermethrin 10 % and Chlorpyrifos 40 %. The ability to dissolve one or more of the aforementioned compounds may then be established and, for each solvent, a profile may be established comprising the dissolution ability for several of the aforementioned compounds.

Hence, an application where NMP, NEP, DMAc, or DMF is suitably used as a solvent, in particular in agricultural applications, at a given concentration could be defined as those applications where one or more, preferably two or more, more preferably three or more, most preferably four or more compounds selected from the group of Alachlor, Propoxur, Oxyfluorfen, Difenoconazole, Trifluralin, Triadimenol, Tebuconazole, Pendimethalin, Propanil, Phenmedipham, Alpha-Cypermethrin and Chlorpyrifos are stably dissolved by NMP, NEP or DMF at room temperature at a given concentration. Of course, other compounds to be dissolved could also be chosen, depending on the application.

In a further aspect, the invention is also concerned with the use of N-n-butylpyrrolidone as a co-solvent in a solvent comprising at least NMP, DMAc, or DMF, i.e. partially replacing NMP, NEP, DMAc, or DMF, or adding N-n-butylpyrrolidone to NMP, NEP, DMAc, or DMF in the application at hand, to improve the toxicological properties of the resulting solvent mixture. More in particular, the invention is concerned with the use of solvent mixtures comprising NMP, NEP, DMAc, or DMF and N-n-butylpyrrolidone, comprising at least 1 vol% of N-n-butylpyrrolidone , preferably at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 vol%, or any range in between two aforementioned values, of N-n-butylpyrrolidone. Most preferably, the solvent mixture comprises at least 50 vol% of N-n-butylpyrrolidone.

In a further aspect, the invention is also concerned with the use of N-n-butylpyrrolidone as a co-solvent in a solvent comprising a second solvent which itself is a suitable replacement for NMP, NEP, DMAc or DMF, i.e. partially replacing the second solvent or adding N-n-butylpyrrolidone to the second solvent in the application at hand. Such second solvent may be one of the solvents currently disclosed in the prior art as a replacement solvent for NMP, such as, but not limited to, the members of the group consisting of N-ethyl-2-pyrrolidone (NEP), 1,5-dimethyl-pyrrolidone (DMP), dipropyleneglycol dimethyl ether (DPGDME), a mixture of ethyl lactate with a methyl ester derived from soya bean oil or corn oil, such as the product commercially offered under the reference ELSOL™ - NMPR, poly(ethylene glycol) dimethyl ether (commonly called "polyglyme", diethylene glycol diethyl ether (commonly called "ethyl diglyme", 1,3-dioxolanes, dimethyl sulphoxide (DMSO) and methyl-5-(dimethylamino)-2-methyl-5-oxopentanoate, such as the commercial product offered as RhodiaSolv® PolarClean. More in particular, the invention is concerned with the use of solvent mixtures comprising said second solvent and N-n-butylpyrrolidone, comprising at least 1 vol% of N-n-butylpyrrolidone, preferably at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 vol%, or any range in between two aforementioned values, of N-n-butylpyrrolidone. Most preferably, the solvent mixture comprises at least 50 vol% of N-n-butylpyrrolidone.

Within the context of this application, when reference is made to pyrrolidone, and unless otherwise explicitly mentioned, reference is made to 2-pyrrolidone, i.e. a pyrrolidone molecule with one nitrogen atom adjacent to the oxy-substituent.

Within the context of this application, when reference is made to the use as a solvent, and unless otherwise explicitly mentioned, reference is made to the use as an inert solvent, i.e. a solvent which does not, or to a minimal extent, react with the one or more chemical compounds which are to be dissolved into the solvent in a given application.

Within the context of this application, when reference is made to the use of a compound as a solvent, and unless otherwise explicitly mentioned, reference is made to said solvent being or behaving as a liquid in the given application, preferably as a liquid at standard temperature and pressure (STP, defined herewith as 25°C and 1 atmosphere = 1,013 bar = 101 325 Pascal).

Within the context of this application, when reference is made to a pyrrolidone compound as a solvent, it is understood that reference is made to quantities of said compounds which are sufficient to be suitable as a solvent. This quantity may range between 10⁻¹² to 10⁺¹² mol, more preferably between laboratory scale amounts and plant scale amounts.

### Chemical properties

Each compound according to the invention is envisioned to be a high boiling, polar, aprotic, organic solvent with a low viscosity, and is completely miscible with water. It is envisioned to be a reusable, non-corrosive solvent, having a high flash point and a low surface tension. It is envisioned to dissolve inorganic compounds such as inorganic salts, it may be used to separate aromatic from aliphatic compounds and is preferably also biologically degradable.

For comparison, the prior art pyrrolidones have been listed in Table 2.

N-n-butylpyrrolidone is either commercially available or may be manufactured according to common chemical knowledge.

A typical manufacturing procedure may consist of a reaction of γ-butyrolactone or appropriately methyl substituted γ-butyrolactone with an amine (such as propylamine, n-butylamine, isobutylamine, t-butylamine, n-pentylamine and methyl-substituted butylamine) under elevated temperature and pressure. The resulting reaction mixture is subsequently purified by distillation.

### Applications

According to one embodiment, N-n-butylpyrrolidone is suitable to be used as a dissolution agent, a dilution agent, an extraction agent, a cleaning agent, a stripping agent, a removing agent, a degreasing agent, an absorption agent, a photoresist stripper and/or a dispersion agent.

The aforementioned properties of N-n-butylpyrrolidonemake N-n-butylpyrrolidonean excellent choice for replacing, partly or wholly, NMP, NEP, DMAc, or DMF as a solvent in a wide range of applications:
(i) The use of N-n-butylpyrrolidone as a solvent in an agrochemical formulation.
   N-n-butylpyrrolidone may be used as an effective solvent or co-solvent acting as a dissolution, dilution or dispersion agent for insecticides, herbicides, fungicides, pesticides, seed treatment products and bioregulators which may often be insoluble in other liquids and which often require polar solvents. Either solvent concentrates or emulsifiable concentrates may require such a solvent, and these may be further formulated with other additional ingredients (surfactants, co-solvents, ...). N-n-butylpyrrolidone may be used on growing crops because of their favourable toxicological profile.
(ii) The use of N-n-butylpyrrolidone as a solvent in cleaning agents.
   Due to the high solvency power for plastics, resins, oils and greases, N-n-butylpyrrolidone may be used for various cleaning purposes, in particular industrial cleaning purposes, in particular in the textile industry for the removal of polymeric materials, dyes and other contaminants. These cleaning purposes include the use as an efficient stripping agent for a varnish, a paint and another finish based on a cellulosic, vinyl, acrylic and/or other resin. Such a solvent contributes to the penetration of the film and exerts a lifting action at the substrate interface. Other examples are the removal of carbon deposits and/or other combustion products from internals of combustion engines.
   Next to industrial uses, N-n-butylpyrrolidone may be used for various household applications such as hard surface cleaners, applications which have been banned in Europe for NMP, NEP, DMAc or DMF because of its reprotoxic properties.
(iii) The use of N-n-butylpyrrolidone as a solvent in polymer manufacturing/processing/deposition.
   N-n-butylpyrrolidone may be an efficient solvent, a dissolution agent, a dilution agent, an extraction agent, an absorption agent and/or a dispersion agent, for polymerization reactions, as well as for coating, spinning, laminating, moulding, extruding and stripping processes. Numerous resins, including many which are insoluble or difficult to dissolve in other solvents, may be dissolved in the pyrrolidones according to the invention. A non-limitative list of such resins would include cellulose derivatives, polyamide, polyimides, polyesters, polystyrene, polyacrylonitrile, polyvinylchloride, polyvinylpyrrolidone, polyvinylacetate, polycarbonates, polyethersulphones, polysulphones, polyethers, polyurethanes, epoxy resins and many copolymers. One might consider the use of N-n-butylpyrrolidone for specific applications such as vinyl coatings, polystyrene- and acrylic-based floor finishes and polishes, spinning acrylic and other synthetic fibres, coating tank interiors with butadiene/acrylonitrile copolymers, extrusion of polyvinylfluoride, nylon moulding, production of PVC sheets and moulded products, paint removers, manufacture of wire insulation enamels and high temperature laminates, preparation of polyurethanes, applying or stripping epoxy coatings, dispersion of pigments in paints and other decorative finishes, rubber and vinyl cements, and many others.
   A specific example of the use of N-n-butylpyrrolidone is in the manufacturing of poly(amides-imide) resins and in the application process of these polymers in the production of wire enamels. In transformers, generators and electric motors the electric insulating material protecting the copper or aluminium wire is a thin coating of a high performance polymer. Adequate thermal, mechanical and electrical properties must be maintained. One such polymer is poly(amide-imide) resin. Reactions for the production of such polymers are carried out in a polar solvent, N-n-butylpyrrolidone being a good candidate for the mentioned purpose, replacing the commonly used NMP or other. The copper or aluminium wires are then pulled through such a polymer solution, during which a polymer coating is deposited onto the wire. This process is repeated a number of times, after which the remaining solvent is evaporated from the coating.
   Another specific example, resembling the above mentioned, is the use of N-n-butylpyrrolidone as a solvent in the manufacturing of a polytetrafluoroethylene polymer (PTFE) and/or the subsequent deposition of any one of such polymers onto a substrate, which may be selected from various substrates (including cooking gear), according to a process similar as described above.
   Another specific example is the manufacturing of batteries, such as lithium ion batteries, wherein a coating is deposited on an electrode, after being dissolved in a solvent such as NMP, NEP, DMAc or DMF.
(iv) The use of N-n-butylpyrrolidone as solvent in carrying out a chemical and/or a pharmaceutical reaction.
   N-n-butylpyrrolidone may be a preferred solvent for carrying out chemical or pharmaceutical reactions, because of their solvency power for many pharmaceuticals or chemical compounds which are difficult to dissolve in other solvents. One might mention examples such as carbonylation reactions, esterification reactions, the preparation of nitriles, fluorination reactions, polymerization reactions and many others.
(v) The use of N-n-butylpyrrolidone as a solvent in microelectronics manufacturing.
   N-n-butylpyrrolidone may be a useful solvent in the microelectronic manufacturing industry for cleaning and degreasing operations, but also in the manufacturing process of a printed circuit board or a microchip where it may be used as a photoresist stripper or stripping agent.
(vi) The use of N-n-butylpyrrolidone in a petrochemical process, such as the extraction of butadiene, acetylene, or another diolefin, conjugated or not, acetylene or not. In such processes, a solvent like NMP, NEP, DMF, DMAc may be used as a selective solvent in extractive distillation processes.

Other potential applications are the use in ink systems, or in several chemical extraction processes such as extraction of aromatics, lube oil or butadiene, gas purification, and in acetylene recovery.

### EXAMPLES:

### Experiment 1: Wire enamel application example

The applicants express their special thanks to Lyonelle Sandjong for her assistance in performing this experiment.

### Preparation of polyamideimide in N-n-butyl-2-pyrrolidone

A five-necked reactor vessel with a volume of 3 litres was equipped with a stirrer, a cooling tube and a reflux condenser. 384 grams (g) of trimellitic anhydride (TMA), 500 g of methylene diphenyl 4,4'-diisocyanate (MDI) and 1200 g of N-n-butyl-2-pyrrolidone (NBP) were introduced into the reactor. The resultant mixture was reacted for 2 hours at 80°C, subsequently heated up to 140°C and kept under stirring at that temperature until no further carbon dioxide was forming. Thereafter, the polymer solution was cooled to 55°C, and 114 g of NBP, 144 g of xylene and 166 g of naphtha solvent were added to the polymer solution. According to the above procedure, a polyamideimide solution having a resin concentration of 34.3 wt.%, a viscosity at 20°C of 2210 mPa.s and an average molecular weight Mw of 8100 g/mole, determined by Gel Permeation Chromatography (GPC) or Size Exclusion Chromatography (SEC) using polystyrene (PS) for the calibration, was obtained.

The polyamideimide was obtained in solution in the solvent N-n-butyl-2-pyrrolidone.

### Comparative example

A polyamideimide in N-methyl-2-pyrrolidone was prepared using the same above procedure by replacing 1200 g of N-n-butyl-2-pyrrolidone by 1200 g of N-methyl-2-pyrrolidone. According to this procedure, a polyamideimide solution having a resin concentration of 34.5 wt.%, a viscosity at 20 °C of 2130 mPa.s and a molecular weight Mw of 14400 g/mole eq. PS was obtained.

### Enamelling and testing:

Copper wires with a bare wire thickness of 0.071 mm were used as conductor of the insulated wires. The enamel was coated onto the wire and baked 16 times in an air-recirculation enamelling machine HRD at a temperature of 650°C at an enamelling speed of 86 m/min. Dies were used as application system. The resulting layer thickness was 0.060 mm.

**Table 1: properties of the enameled copper wires**

| | Comparative example (NMP) | Example (NBP) |
|---|---|---|
| Tangent Delta (°C) | 268 | 270 |
| Heat shock (30 @ 220 °C, % pre-stretching) | 20 | 20 |
| Flexibility (1D, % pre-stretching) | 20 | 20 |

From these tests, it may be concluded that the polyamideimide produced and dissolved in N-n-butyl-2-pyrrolidone could be enamelled and the resulted enamelled copper wires exhibits properties similar to a polyamideimide produced and dissolved in N-methyl-2-pyrrolidone.

### Reprotoxicitv

### OECD 422

The reprotoxicity was tested in a method, similar to the prescriptions in OECD 422 "Guideline for the testing of chemicals, Combined Repeated Dose Toxicity Study with the Reproduction/Development Toxicity Screening Test", March 22, 1996. According to this test, the method comprises administering (preferably orally) the test substance in graduated doses to several groups of male and female rats which are allowed to mate. Males in the test are to be dosed for a minimum of four weeks, up to and including the day before scheduled kill, including a minimum of two weeks prior to mating, during the mating period and, approximately, two weeks post mating. The combination of a pre-mating dosing period of two weeks and subsequent mating and fertility observations with an overall dosing period of at least four weeks, followed by a detailed histopathology of the male gonads, is considered sufficient to enable detection of the majority of effects on male fertility and spermatogenesis. Females are to be dosed throughout the study.

As demonstrated further below, N-n-butylpyrrolidone did not show any evidence of reprotoxicity when tested according to the OECD 422 Guidelines, performed on Han Wistar rats. The tests described herein below were however somewhat more limited, as they are screening tests, leading into a subsequent and more comprehensive study.

### Experiment 2

### 2.1 General

The purpose of this study was to detect effects of 4 test items or compounds on the development of the embryo and foetus consequent to exposure of the female Han Wistar rat to the test items from day 6 post coitum (implantation) to day 20 post coitum (the day prior to Caesarean section). Particular attention was given to the foetal skeleton. Each group received one test compound, at a dose level of 100 or 500 mg/kg/day. The results were compared to a negative control group, which were administered distilled water (Aqua dest.).

Each group consisted of 5 mated females, treated by gavage, once daily as follows:

| | |
|---|---|
| Test Item: Group 1: | Aqua dest. (negative control) |
| Group 2: | N-n-butylpyrrolidone |
| Group 3: | N-n-propylpyrrolidone |
| Group 4: | N-isobutylpyrrolidone |
| Group 5: | N-isopropylpyrrolidone |
| | |
| Dose Levels: Groups 3, 5 | 500 mg/kg body weight/day |
| Groups 2, 4 | 100 mg/kg body weight/day |

A standard dose volume of 5 mL/kg body weight with a daily adjustment to the actual body weight was used.

### 2.2 Findings summary

### 2.2.1 Maternal Data

### MORTALITY AND GENERAL TOLERABILITY

All females survived until the scheduled necropsy

Bedding in the mouth was observed in one female in group 2 (N-n-butylpyrrolidone) and in all females in group 3 (N-n-propylpyrrolidone) during the treatment period. This was considered as being a sign of discomfort rather than a toxic effect of the test item.

In group 5 (N-Isopropylpyrrolidone), 4 dams had bedding in the mouth after dosing. This was considered a reaction to treatment with the test item or compound.

No further clinical signs were observed in any female in any dose group.

### FOOD CONSUMPTION

In group 2, the mean food consumption was not considered to have been affected by treatment with the test item or compound.

In group 3, the food consumption was statistically significantly reduced at the start of treatment over days 6 - 9 post coitum (p.c.). Thereafter, mean food consumption was similar to that of the control group. Over the treatment period, days 6 - 21 p.c., mean food consumption was -9.1% compared to the control group. The reduction was considered to be a test item-related effect.

In group 4, the mean food consumption was similar to that of the control group throughout the study.

In group 5 (N-Isopropylpyrrolidone), the mean food consumption decreased slightly at treatment start over days 6 - 9 but recovered thereafter.

### BODY WEIGHTS

In group 2, the mean body weight, the body weight gain and the corrected body weight gain were similar to that of the control group.

In group 3, the body weight gain was statistically significantly reduced from day 8 p.c. until the end of the study, although absolute body weight was at no time statistically significantly reduced. Over the treatment period, the body weight gain was 36% compared to 49% in the control group. Corrected body weight gain was reduced without statistical significance (7.6% compared to 11.1% in the control group).

In group 4, the mean body weight, the body weight gain and the corrected body weight gain were not affected by treatment with the test item or compound.

In group 5 (N-Isopropylpyrrolidone), the mean body weight gain was statistically significantly reduced but this did not have a clear effect on absolute body weight. The corrected body weight gain was reduced but not statistically significantly.

### MACROSCOPICAL FINDINGS

At necropsy, no relevant findings were observed in any female in any group.

### 2.2.2 Foetal Data

The foetuses were removed from the uterus, sexed, weighed individually, examined for gross external abnormalities, sacrificed by a subcutaneous injection of sodium pentobarbital, eviscerated and with the exception of over the paws, the skin was removed and discarded. Carcasses were processed through solutions of ethanol, glacial acetic acid with Alcian blue (for cartilage staining), potassium hydroxide with Alizarin red S (for clearing and staining ossified bone) and aqueous glycerine for preservation and storage. The skeletons were examined and all abnormal findings and variations were recorded.

### EXTERNAL ABNORMALITIES AND VARIATIONS

No test item-related findings were observed in any litter in any group.

### SEX RATIOS

The sex ratios of the foetuses were not affected by treatment with the test item in any group.

### BODY WEIGHTS

In groups 3 and 5, the weights of the foetuses were statistically significantly reduced in both the male and female foetuses and were outside the range of the historical control data. This reduction was considered to be a test item-related effect.

In groups 2 and 4 the foetal weights were not affected by treatment with the test item.

### BONE AND CARTILAGE ABNORMALITIES AND VARIATIONS / OSSIFICATION AND SUPERNUMERARY RIBS

In groups 2 and 4, no test item-related findings were observed.

In group 3 (N-n-propylpyrrolidone), there was an increased incidence of zygomatic arch fusion in the skull. It could not be excluded that this was due to the treatment with the test item.

Further in group 3 (N-n-propylpyrrolidone), there was a slightly increased incidence of non-ossified cervical vertebral bodies and supernumerary ribs.

In group 5 (N-Isopropylpyrrolidone), there was an increased incidence of cervical ribs. Although these ribs had no distal cartilage and may result in non-permanent structures, the high incidence may suggest an indication of a slight disturbance in the configuration of the axial skeleton. In addition, the incidence of zygomatic arch fusion as well as incompletely ossified cranial structures was slightly increased.

### 2.2 Findings summary

### 2.3 Conclusion

In group 2 (N-n-butylpyrrolidone, 100 mg/kg body weight/day), bedding in the mouth was observed in one female. No effects on food consumption or body weight were observed. The weights of the foetuses were not affected by treatment with the test item. No test item-related effects were observed in the foetuses.

In group 3 (N-n-propylpyrrolidone, 500 mg/kg body weight/day), bedding in the mouth was observed in all females. Food consumption was statistically significantly reduced at the start of treatment. Body weight gain was statistically significantly reduced for most of the study and corrected body weight gain was reduced without statistical significance. The weights of the foetuses were statistically significantly reduced in both the males and females and were outside the range of the historical control data. There was an increased incidence of zygomatic arch fusion in the skull and a slightly increased incidence of non-ossified cervical vertebral bodies and supernumerary ribs in the foetuses.

In group 4 (N-isobutylpyrrolidone, 100 mg/kg body weight/day), no effects on food consumption or body weight were observed. The weights of the foetuses were not affected by treatment with the test item. No test item-related effects were observed in the foetuses.

In group 5 (N-Isopropylpyrrolidone), 4 dams had bedding in the mouth after dosing. Mean food consumption and body weight were similar to that of the control group. Body weight gain was statistically significantly reduced and corrected body weight gain was reduced but not statistically significantly. Post-implantation loss and embryonic resorptions were increased and the number of foetuses per dam decreased. The mean weight of the foetuses on a litter and a foetus basis were statistically significantly reduced. During foetal examination, a clearly increased incidence of cervical ribs was observed. This high incidence may suggest an indication of a slight disturbance in the configuration of the axial skeleton. The number of incompletely ossified cranial structures was slightly increased, indicating retardation in the skeletal development.

**Table 2: Pyrrolidone compounds according to the prior art**

| | NMP¹ | DMP² | NEP |
|---|---|---|---|
| CAS-No. | 872-50-4 | | 2687-91-4 |
| Formula | C5H9NO | C6H11 NO | C6H11 NO |
| MWt (g/mol) | 99,13 | 113,16 | 113,16 |
| Colour | colourless | colourless | colourless |
| Boiling point (°C) | 202 | 215-217 | 212,5 |
| Flash point (°C) | 91 | 89 | 90,8 |
| Density (g/cm³) at 25 °C | 1,028 | 0.982 | 0,998 |
| Surface tension (mN/m) | 40,7 | - | 69 |
| Viscosity (mPa.s) at 25 °C | 1,66 | - | 2,1 |
| Polarity | - | - | |
| Reactivity | - | - | |
| Biodegradability | 90% - readily biodegradable | - | |
| Miscibility with water | fully | - | fully |
| Toxicity | LD50 oral rat 3914 mg/kg | Skin and serious eye irritation | LD50 oral rat 3200 mg/kg |
| Reprotoxicity | reprotoxic (Cat2) | Screening test indicates reprotox potential | Reprotoxic (Cat2) |

| | | | |
|---|---|---|---|
| 1: Safety Data Sheet Sigma-Aldrich, 19.08.2011 2: Safety Data Sheet Sigma-Aldrich, 28.07.2010. | | | |

## Claims

1. Use of N-n-butylpyrrolidone as a non-reprotoxic solvent.

2. The use according to claim 1 for the partial or complete replacement of a solvent selected from the list consisting of N-methylpyrrolidone (NMP), N-ethyl-2-pyrrolidone (NEP), dimethyl formamide (DMF), N,N-dimethyl acetamide (DMAc), and mixtures thereof as a solvent whereby the solvent comprises at least 1 vol% of N-n-butylpyrrolidone.

3. The use according to any preceding claim as a co-solvent in a solvent comprising a second solvent which is a replacement solvent for NMP, NEP, DMAc, or DMF, preferably selected from the group consisting of 1,5-dimethyl-pyrrolidone (DMP), dipropyleneglycol dimethyl ether (DPGDME), a mixture of ethyl lactate with a methyl ester derived from soya bean oil or corn oil, poly(ethylene glycol) dimethyl ether (commonly called "polyglyme"), diethylene glycol diethyl ether (commonly called "ethyl diglyme"), 1,3-dioxolanes, dimethyl sulphoxide (DMSO) and methyl-5-(dimethylamino)-2-methyl-5-oxopentanoate, wherein the solvent comprises at least 1 vol% of N-n-butylpyrrolidone.

4. The use according to any one of the preceding claims, wherein the solvent is used as a dissolution agent, a dilution agent, an extraction agent, a cleaning agent, a stripping agent, a removing agent, a degreasing agent, an absorption agent and/or a dispersion agent.

5. The use according to claim 4, wherein the solvent is used as a dissolution, dilution or dispersion agent in an agrochemical formulation.

6. The use according to claim 4, wherein the solvent is used as a stripping agent for a varnish, a paint and/or another finish based on a cellulosic, vinyl, acrylic and/or other resin.

7. The use according to claim 4, wherein the solvent is used as a removal agent of carbon deposits and other combustion products from internals of combustion engines.

8. The use according to claim 4, wherein the solvent is used as a cleaning agent for the removal of polymeric materials, dyes and other contaminants.

9. The use according to claim 4, wherein the solvent is used as a dissolution agent, a dilution agent, an extraction agent, an absorption agent and/or a dispersion agent for polymerization reactions, as well as for coating, spinning, laminating, moulding, extruding and stripping processes.

10. The use according to claim 4, wherein the solvent is used as a dissolution agent, a dilution agent, an extraction agent, an absorption agent, a reaction medium, and/or a dispersion agent in the manufacturing of a resin selected from the group consisting of a cellulose derivative, a polyamide, a polyimide, a polyester, a polystyrene, a polyacrylonitrile, a polyvinylchloride (PVC), a polyvinylpyrrolidone, a polyvinylacetate, a polycarbonate, a polyethersulphone, a polysulphone, a polyether, a polyurethane, a polyesterimide, an epoxy resin, a poly(amide-imide) resin, and many copolymers thereof, and in the application process of any of these polymers in the production of a wire enamel.

11. The use according to claim 4, wherein the solvent is used as a dissolution agent, a dilution agent, an extraction agent, an absorption agent a reaction medium, and/or a dispersion agent in the manufacturing of a polytetrafluoroethylene polymer and/or the subsequent deposition of any one of such polymers onto a substrate.

12. The use according to claim 4, wherein the solvent is used as a dissolution agent, a dilution agent, an extraction agent, an absorption agent a reaction medium, and/or a dispersion agent for carrying out a chemical or a pharmaceutical reaction.

13. The use according to claim 4, wherein the solvent is used in the microelectronics manufacturing industry, including as a photoresist stripping agent in the manufacturing process of a printed circuit board and/or a microchip.

14. A solvent comprising N-methylpyrrolidone (NMP) and at least 1 vol% of N-n-butylpyrrolidone.

15. A solvent comprising a second solvent which is a replacement solvent for N-methylpyrrolidone (NMP), selected from the group of N-ethyl-2-pyrrolidone (NEP), 1,5-dimethyl-pyrrolidone (DMP), dipropyleneglycol dimethyl ether (DPGDME), a mixture of ethyl lactate with a methyl ester derived from soya bean oil or corn oil, poly(ethylene glycol) dimethyl ether (commonly called "polyglyme"), diethylene glycol diethyl ether (commonly called "ethyl diglyme"), 1,3-dioxolanes, dimethyl sulphoxide (DMSO) and methyl-5-(dimethylamino)-2-methyl-5-oxopentanoate and as a first solvent at least 1 vol% of N-n-butylpyrrolidone.

## Patentansprüche

1. Verwendung von N-n-Butylpyrrolidon als ein nicht-reproduktionstoxisches Lösungsmittel.

2. Die Verwendung nach Anspruch 1 für den teilweisen oder kompletten Ersatz eines Lösungsmittels ausgewählt aus der Liste bestehend aus N-Methylpyrrolidon (NMP), N-Ethyl-2-Pyrrolidon (NEP), Dimethylformamid (DMF), N,N-Dimethylacetamid (DMAc), und Mischungen davon als ein Lösungsmittel, wobei das Lösungsmittel mindestens 1 Vol.-% N-n-Butylpyrrolidon enthält.

3. Die Verwendung nach irgendeinem vorigen Anspruch als ein Co-Lösungsmittel in einem Lösungsmittel, welches ein zweites Lösungsmittel enthält, das ein Ersatzlösungsmittel für NMP, NEP, DMAc, oder DMF ist, bevorzugt ausgewählt aus der Gruppe bestehend aus 1,5-Dimethylpyrrolidon (DMP), Dipropylenglycoldimethylether (DPGDME), einer Mischung von Ethyllactat mit einem Methylester abgeleitet von Sojabohnenöl oder Maisöl, Poly(ethylenglycol)-Dimethylether (allgemein "Polyglym" genannt), Diethylenglycol-Diethylether (allgemein "Ethyl-Diglym" genannt), 1,3-Dioxolanen, Dimethylsulfoxid (DMSO) und Methyl-5-(dimethylamin)-2-methyl-5-oxopentanoat, wobei das Lösungsmittel mindestens 1 Vol.-% N-n-Butylpyrrolidon enthält.

4. Die Verwendung nach irgendeinem der vorigen Ansprüche, wobei das Lösungsmittel als ein Auflösungsmittel, ein Verdünnungsmittel, ein Extraktionsmittel, ein Reinigungsmittel, ein Abziehmittel, ein Entfernungsmittel, ein Entfettungsmittel, ein Absorptionsmittel und/oder ein Dispergiermittel verwendet wird.

5. Die Verwendung nach Anspruch 4, wobei das Lösungsmittel als ein Auflösungs-, Verdünnungs- oder Dispergiermittel in einer agrochemischen Rezeptur verwendet wird.

6. Die Verwendung nach Anspruch 4, wobei das Lösungsmittel als ein Abziehmittel gegen einen Lack, einen Anstrich und/oder eine andere Oberflächenbehandlung auf Grundlage eines Cellulose-, Vinyl-, Acryl- und/oder anderen Harzes verwendet wird.

7. Die Verwendung nach Anspruch 4, wobei das Lösungsmittel als ein Entfernungsmittel gegen Kohlenstoffablagerungen und anderen Verbrennungsprodukten von Einbauteilen von Verbrennungsmotoren verwendet wird.

8. Die Verwendung nach Anspruch 4, wobei das Lösungsmittel als ein Reinigungsmittel für die Entfernung von polymeren Materialien, Farben und anderen verunreinigenden Substanzen verwendet wird.

9. Die Verwendung nach Anspruch 4, wobei das Lösungsmittel als ein Auflösungsmittel, ein Verdünnungsmittel, ein Extraktionsmittel, ein Absorptionsmittel und/oder ein Dispergiermittel für Polymerisationsreaktionen sowie für Beschichtungs-, Spinn-, Laminier-, Form-, Extrusions- und Strippungsvorgänge verwendet wird.

10. Die Verwendung nach Anspruch 4, wobei das Lösungsmittel als ein Auflösungsmittel, ein Verdünnungsmittel, ein Extraktionsmittel, ein Absorptionsmittel, ein Reaktionsmedium und/oder ein Dispergiermittel in der Herstellung eines Harzes, ausgewählt aus der Gruppe bestehend aus einem Cellulosederivat, einem Polyamid, einem Polyimid, einem Polyester, einem Polystyrol, einem Polyacrylnitril, einem Polyvinylchlorid (PVC), einem Polyvinylpyrrolidon, einem Polyvinylacetat, einem Polycarbonat, einem Polyethersulfon, einem Polysulfon, einem Polyether, einem Polyurethan, einem Polyesterimid, einem Epoxidharz, einem Poly(amid-imid)harz, und vielen Copolymeren davon, und im Anwendungsverfahren irgendeines dieser Polymere in der Herstellung eines Drahtlackes verwendet wird.

11. Die Verwendung nach Anspruch 4, wobei das Lösungsmittel als ein Auflösungsmittel, ein Verdünnungsmittel, ein Extraktionsmittel, ein Absorptionsmittel, ein Reaktionsmedium und/oder ein Dispergiermittel in der Herstellung eines Polytetrafluorethylenpolymers und/oder der nachfolgenden Ablagerung irgendeines solchen Polymers auf einem Substrat verwendet wird.

12. Die Verwendung nach Anspruch 4, wobei das Lösungsmittel als ein Auflösungsmittel, ein Verdünnungsmittel, ein Extraktionsmittel, ein Absorptionsmittel, ein Reaktionsmedium, und/oder ein Dispergiermittel zur Ausführung einer chemischen oder einer pharmazeutischen Reaktion verwendet wird.

13. Die Verwendung nach Anspruch 4, wobei das Lösungsmittel in der Mikroelektronikherstellungsindustrie verwendet wird, einschließlich als Fotolack-Abziehmittel im Herstellungsverfahren einer Leiterplatte und/oder eines Mikrochips.

14. Ein Lösungsmittel, das N-Methylpyrrolidon (NMP) und mindestens 1 Vol.-% N-n-Butylpyrrolidon enthält.

15. Ein Lösungsmittel, welches ein zweites Lösungsmittel enthält, das ein Ersatzlösungsmittel für N-Methylpyrrolidon (NMP) ist, ausgewählt aus der Gruppe bestehend aus N-Ethyl-2-Pyrrolidon (NEP), 1,5-Dimethylpyrrolidon (DMP), Dipropylenglycoldimethylether (DPGDME), einer Mischung von Ethyllactat mit einem Methylester abgeleitet von Sojabohnenöl oder Maisöl, Poly(ethylenglycol)-Dimethylether (allgemein "Polyglym" genannt), Diethylenglycol-Diethylether (allgemein "Ethyl-Diglym" genannt), 1,3-Dioxolanen, Dimethylsulfoxid (DMSO) und Methyl-5-(dimethylamin)-2-methyl-5-oxopentanoat, und als ein erstes Lösungsmittel mindestens 1 Vol.-% N-n-Butylpyrrolidon.

## Revendications

1. Utilisation de la N-n-butylpyrrolidone comme un solvant reprotoxique.

2. Utilisation selon la revendication 1 pour le remplacement partiel ou total d'un solvant sélectionné parmi la liste constituée de N-méthylpyrrolidone (NMP), N-éthyl-2-pyrrolidone (NEP), diméthyl-formamide (DMF), N,N-diméthyl acétamide (DMAc), et des mélanges de ceux-ci comme un solvant par lequel le solvant comprend au moins 1 % en volume de N-n-butylpyrrolidone.

3. Utilisation selon l'une quelconque des revendications précédentes comme un co-solvant dans un solvant comprenant un deuxième solvant qui est un solvant de remplacement pour NMP, NEP, DMAc, ou DMF, sélectionné de préférence parmi le groupe constitué de 1,5-diméthyl-pyrrolidone (DMP), dipropylèneglycol diméthyl éther (DPGDME), un mélange de lactate d'éthyle avec un ester de méthyle dérivé de l'huile de soja ou huile de maïs, poly(éthylène glycol) diméthyl éther (communément appelé « polyglyme »), diéthylène glycol diéthyl éther (communément appelé « éthyl diglyme »), 1,3-dioxolanes, diméthyl sulfoxyde (DMSO) et 5-(diméthylamino)-2-méthyl-5-oxo-pentanoate de méthyle, dans laquelle le solvant comprend au moins 1 % en volume de N-n-butylpyrrolidone.

4. Utilisation selon l'une quelconque des revendications précédentes, dans laquelle le solvant est utilisé comme un agent de dissolution, un agent de dilution, un agent d'extraction, un agent de nettoyage, un agent de décapage, un agent d'enlévement, un agent de dégraissage, un agent d'absorption et/ou un agent de dispersion.

5. Utilisation selon la revendication 4, dans laquelle le solvant est utilisé comme un agent de dissolution, de dilution ou de dispersion dans une formulation agrochimique.

6. Utilisation selon la revendication 4, dans laquelle le solvant est utilisé comme un agent de décapage pour un vernis, une peinture et/ou autre finition à base d'une résine cellulosique, vinylique, acrylique et/ou d'une autre résine.

7. Utilisation selon la revendication 4, dans laquelle le solvant est utilisé comme un agent d'élimination de dépôts de carbone et d'autres produits de combustion à partir des parties internes des moteurs à combustion.

8. Utilisation selon la revendication 4, dans laquelle le solvant est utilisé comme un agent de nettoyage pour éliminer les matériaux polymères, les colorants et d'autres contaminants.

9. Utilisation selon la revendication 4, dans laquelle le solvant est utilisé comme un agent de dissolution, un agent de dilution, un agent d'extraction, un agent d'absorption et/ou un agent de dispersion pour les réactions de polymérisation, ainsi que pour les procédés de revêtement, de filage, de stratification, de moulage, d'extrusion et de décapage.

10. Utilisation selon la revendication 4, dans laquelle le solvant est utilisé comme un agent de dissolution, un agent de dilution, un agent d'extraction, un agent d'absorption, un milieu réactionnel, et/ou un agent de dispersion dans la fabrication d'une résine sélectionnée parmi le groupe constitué d'un dérivé cellulosique, d'un polyamide, d'un polyimide, d'un polyester, d'un polystyrène, d'un polyacrylonitrile, d'un polychlorure de vinyle (PVC), d'un polyvinylpyrrolidone, d'un polyacétate de vinyle, d'un polycarbonate, d'une polyéthersulfone, d'une polysulfone, d'un polyéther, d'un polyuréthane, d'un polyesterimide, d'une résine époxy, d'une résine poly(amide-imide), et des nombreux copolymères de ceux-ci, et dans le procédé d'application de l'un quelconque de ces polymères dans la production d'un émail pour fil métallique.

11. Utilisation selon la revendication 4, dans laquelle le solvant est utilisé comme un agent de dissolution, un agent de dilution, un agent d'extraction, un agent d'absorption, un milieu réactionnel, et/ou un agent de dispersion dans la fabrication d'un polymère de polytétrafluoroéthylène et/ou le dépôt ultérieur de l'un quelconque de ces polymères sur un substrat.

12. Utilisation selon la revendication 4, dans laquelle le solvant est utilisé comme un agent de dissolution, un agent de dilution, un agent d'extraction, un agent d'absorption, un milieu réactionnel, et/ou un agent de dispersion pour effectuer une réaction chimique ou pharmaceutique.

13. Utilisation selon la revendication 4, dans laquelle le solvant est utilisé dans l'industrie de fabrication micro-électronique, notamment comme un agent de décapage de résine photosensible dans le procédé de fabrication d'une carte de circuit imprimé et/ou d'une micropuce.

14. Solvant comprenant de la N-méthylpyrrolidone (NMP) et au moins 1 % en volume de N-n-butylpyrrolidone.

15. Solvant comprenant un deuxième solvant qui est un solvant de remplatcement pour la N-méthylpyrrolidone (NMP), sélectionné parmi le groupe de N-éthyl-2-pyrrolidone (NEP), 1,5-diméthyl-pyrrolidone (DMP), dipropylèneglycol diméthyl éther (DPGDME), un mélange de lactate d'éthyle avec un ester de méthyle dérivé de l'huile de soja ou huile de maïs, poly(éthylène glycol) diméthyl éther (communément appelé « polyglyme »), du diéthylène glycol diéthyl éther (communément appelé « éthyl diglyme »), 1,3-dioxolanes, diméthyl sulfoxyde (DMSO) et 5-(diméthylamino)-2-méthyl-5-oxopentanoate de méthyle et comme un premier solvant au moins 1 % en volume de N-n-butylpyrrolidone.
